# EUROPEAN PATENT APPLICATION

(11) **EP 2 689 738 A1**
(43) Date of publication of application: **29.01.2014**
(21) Application number: 13178275.7
(22) Date of filing: 26.07.2013
(51) Int. Cl.: A61B 18/12, A61B 18/14

(54) **Electromagnetic shielding for an electrosurgical unit**

(30) Priority: 27.07.2012 US 201261676695 P
(71) Applicant: Netherton, Brett Lane, Proserity, SC 29127 (US)
(72) Inventor: Netherton, Brett Lane, Proserity, SC 29127 (US)
(74) Representative: Hargreaves, Timothy Edward

(57) **Abstract**

An electromagnetic shield apparatus is provided for an existing electrosurgical unit (ESU) including a pencil assembly and return pad assembly. The pencil assembly includes a pencil, pencil-assembly cable and terminal couplable to the ESU, and the return pad assembly includes a return pad, return-pad-assembly cable and return-pad-assembly terminal couplable to the ESU. The electromagnetic shield apparatus includes an electrically-conductive shield, a low-impedance, electrically-passive termination jumper and an insulated, low-impedance conductive cable. The electrically-conductive shield is configured for placement over the pencil-assembly cable to thereby cover and extend coaxially with the pencil-assembly cable. The termination jumper is couplable to and between the return-pad-assembly terminal and ESU, and the conductive cable is coupled to and between the electrically-conductive shield and termination jumper.

## Description

### CROSS-REFERENCE TO RELATED APPLICATION(S)

The present application claims priority to U.S. Provisional Patent Application No. 61/676,695, entitled: *Electromagnetic Shielding for an Electrosurgical Unit,* filed on July 27, 2012, the content of which is incorporated herein by reference in its entirety.

### TECHNOLOGICAL FIELD

The present disclosure relates generally to electrosurgical units and, in particular, to an electromagnetic shielding for an electrosurgical unit.

### BACKGROUND

The use of electric current in medical procedures to affect tissue is ubiquitous and has been used for many decades, such as in the case of electrosurgical procedures, electrocauterization procedures and the like. These procedures often employ an electrosurgical unit (ESU) that uses electrical energy created by an ESU generator to heat tissue at a surgical site and limit blood loss. Because of the ease of use and limited blood loss benefits of the ESU, almost all surgeries that create open wound sites utilize the ESU.

The ESU typically functions by using a small surface area patient contact tip (sometimes referred to as the ESU pencil tip) and a large surface area return pad. The surgeon touches the ESU pencil tip at the desired location and activates the ESU, cutting or coagulating the tissue. In other similar procedures, the ESU may be used to desiccate, fulgurate or cauterize tissue.

The ESU is not without dangers, including electrical injury to the patient, the most common being unintentional electrothermal tissue bums. These unintentional electrothermal tissue bums may occur at locations other than the tip of the ESU. One example of unintentional electrothermal tissue bums can occur with patient attachment electrodes such as neurodiagnostic subdermal needle electrodes.

Intraoperative neuromonitoring (IONM) is performed in surgeries where various neural or vascular tissues may be at risk during the surgical procedure. It is not uncommon to place multiple attachment electrodes on the patient for purposes of electrical stimulation or electrical recording of physiologic events. These patient attachment electrodes may be cutaneous electrodes, surface electrodes, subdermal needle electrodes (SDNEs), intramuscular needle electrodes or the like. The surface area of these attachment electrodes is variable, but in some instances such as the use of SDNEs, the surface are of the electrode in contact with patient tissue may be small. These electrodes are typically attached to the patient using a conductive, insulated wire that connects the electrode to the neurodiagnostic stimulating and recording electrical instrumentation. And the electrode wires are sometimes referred to as electrode leadwires.

It is possible to electromagnetically couple the energy from the ESU pencil cable to the electrode leadwires. This electromagnetic coupling may take place through air, although not always through air alone. In the operating room, the space around the patient on the operating room is often filled. One or more surgeons, nurses, surgical scrub technologists, anesthesia staff and or other personnel are often standingvery closely to the patient during the surgery. Other wires, cables, warming blankets, fluid (often conductive fluid) filled bags or containers are just a few other items that may be in close proximity to the patient. In some instances, these nearby items such as these mentioned may represent a low electrical impedance pathway making possible electromagnetic coupling between the ESU pencil cable and the electrode leadwire.

The electromagnetic coupling between the ESU pencil cable and electrode leadwires often occurs on a near continual basis, but frequently does not induce unwanted patient electrothermal bums. But electrothermal bums do occur at patient electrode sites and have been documented in the scientific literature. When these electrothermal bums do occur at SNDE sites, the largest tissue injury often is located subdermally, only leaving a tiny ring of tissue damage at the surface of skin that is discolored. While operating room personnel are typically very attentive to patient skin injury, the operating room is a very busy environment during and post-surgery and patient bums are not always easy to spot. Overlooked or missed patient electrothermal tissue bums may result.

It is clearly desirable to decrease the risk for electrical tissue injury at patient tissue attachment sites not related to the ESU.

### BRIEF SUMMARY

In view of the foregoing, example implementations of the present disclosure are generally directed to an electromagnetic shielding for an electrosurgical unit to decrease the risk of electrical tissue injury. According to one example implementation, an electromagnetic shield apparatus is provided for an existing electrosurgical unit (ESU) including a pencil assembly and return pad assembly. According to this example, the pencil assembly includes a pencil, pencil-assembly cable and terminal couplable to the ESU, and the return pad assembly includes a return pad, return-pad-assembly cable and return-pad-assembly terminal couplable to the ESU.

The electromagnetic shield apparatus may include an electrically-conductive shield, a low-impedance, electrically-passive termination jumper and an insulated, low-impedance conductive cable. The electrically-conductive shield is configured for placement over the pencil-assembly cable to thereby cover and extend coaxially with the pencil-assembly cable. The termination jumper is couplable to and between the return-pad-assembly terminal and ESU, and the conductive cable is coupled to and between the electrically-conductive shield and termination jumper. According to this example, the electrically-conductive shield may be configured to reduce electromagnetic coupling of the pencil-assembly cable and a leadwire in proximity thereto.

According to another example implementation, a pencil assembly is provided for an ESU. The pencil assembly includes a pencil, a cable coupled to the pencil, and a terminal coupled to the cable and couplable to the ESU. The cable includes an insulator surrounding an inner conductor, and further includes an electrically-conductive shielding layer covering the insulator and inner conductor. The terminal includes a plurality of pins receivable by respective receptacles of the ESU, with the electrically-conductive shielding layer being coupled to one of the pins receivable by a respective one of the receptacles of the ESU. In this regard, the respective one of the receptacles of the ESU is coupled by a low-impedance conductive connection to a return input of the ESU at which a return pad assembly is couplable. Similar to before, according to this example, the electrically-conductive shielding layer may be configured to reduce electromagnetic coupling of the cable and a leadwire in proximity thereto.

And according to yet another example implementation, an electromagnetic shield apparatus is provided for an ESU having a return pad assembly including a return pad, return-pad-assembly cable and return-pad-assembly terminal couplable to the ESU. The electromagnetic shield apparatus includes a pencil assembly with a pencil, a pencil-assembly cable coupled to the pencil and a pencil-assembly terminal coupled to the cable and couplable to the ESU. The pencil-assembly cable includes an insulator surrounding an inner conductor, and further includes an electrically-conductive shielding layer covering the insulator and inner conductor.

The electromagnetic shield apparatus of this example also includes a low-impedance, electrically-passive termination jumper couplable to and between the return-pad-assembly terminal and ESU. And the apparatus includes an insulated, low-impedance conductive cable coupled to and between the electrically-conductive shielding layer and termination jumper. And again, the electrically-conductive shielding layer may be configured to reduce electromagnetic coupling of the pencil-assembly cable and a leadwire in proximity thereto.

The features, functions and advantages discussed herein may be achieved independently in various example implementations or may be combined in yet other example implementations further details of which may be seen with reference to the following description and drawings.

### BRIEF DESCRIPTION OF THE DRAWING(S)

Having thus described the technological field of the present disclosure in general terms, reference will now be made to the accompanying drawings, which are not necessarily drawn to scale, and wherein:
FIGS. 1 and 2 are illustrations of a system including a typically-configured electrosurgical generator unit for performing surgery on a patient;
FIGS. 3 and 4 are illustrations of a system including an electrosurgical generator unit and an electromagnetic shield apparatus according to one example implementation;
FIGS. 5 and 6 are illustrations of systems including an electrosurgical generator unit and an electromagnetic shield apparatus according to other example implementations; and
FIG. 7 is an illustration of an electrosurgical generator unit pencil according to one example implementation.

### DETAILED DESCRIPTION

Some implementations of the present disclosure will now be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art. Like reference numerals refer to like elements throughout.

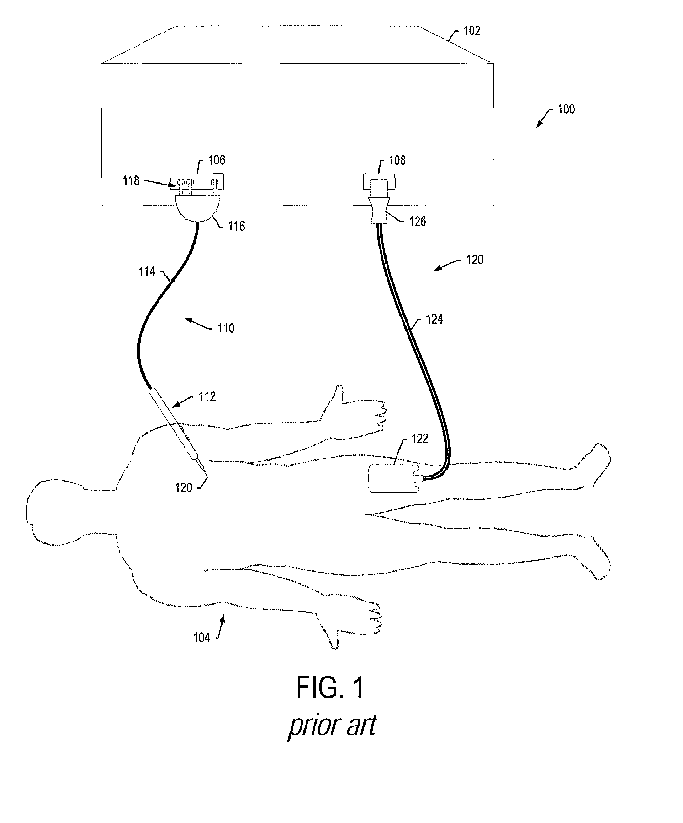

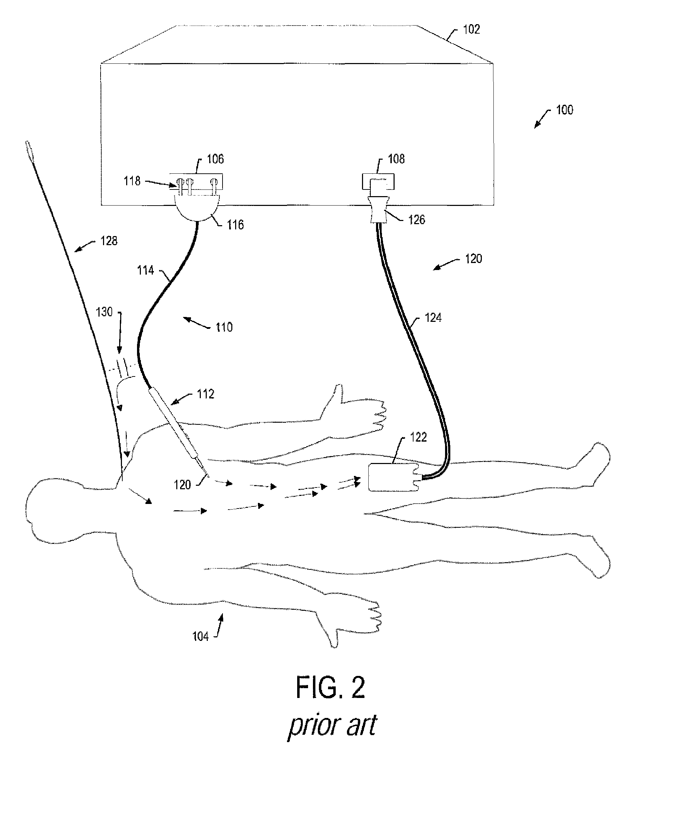

FIGS. 1 and 2 illustrate a system **100** including a typically-configured electrosurgical generator unit (ESU) **102** for performing surgery on a patient **104.** The ESU is generally configured to generate a high-voltage output current. Although described primarily in the context of electrosurgery, the ESU may be used in any of a number of different medical procedures such as electrosurgical procedures, electrocauterization procedures and the like. In these and other similar procedures, the ESU generally serves to cut, coagulate, desiccate, fulgurate or cauterize tissue during many different portions of a procedure. It operates by sending pulses of a particular frequency through a circuit that includes the patient. In some examples such as those in which the ESU is used to cut tissue, the frequency used may be relatively low, such as in the range of 1,300 - 1,500 Hz. In other examples such as those in which the ESU is used to coagulate tissue, the frequency used may be well over 100,000 Hz.

As shown, the ESU includes terminal connections for an active output **106** and return input **108.** The active output serves to connect an active side **110** of the circuit to the ESU **102,** which is responsible for cutting tissue. As shown, the active side may include an ESU pencil assembly including a pencil **112,** pencil-assembly cable **114** and pencil-assembly terminal **116.** The pencil typically includes two activation buttons, one for coagulation and another for cutting. The pencil-assembly cable includes an insulator over one or more internal conductors configured to carry current generated by the ESU. The pencil-assembly cable is typically six feet or longer and serves to allow the ESU to sit away from the operating room table. The pencil-assembly terminal allows for termination of the pencil assembly with the ESU. In this regard, the pencil-assembly terminal typically includes a plurality of (e.g., three) pins **118,** and the active output includes a plurality of receptacles for receiving respective ones of the pins, to support cautery as well as electrosurgical functions.

The ESU pencil assembly is configured for cutting tissue at a tip **120** of the pencil **112.** The pencil tip has a small surface area creating a high current density at the tip. When the ESU **102** is activated, the current density is large enough that it actually creates an arc between the tip of the pencil and tissue, thereby cutting or cauterizing the tissue.

The return input **108** serves to connect a return side **120** of the circuit to the ESU **102.** As shown, the return side may include an ESU return pad assembly including a return pad **122,** return-pad-assembly cable **124** and return-pad-assembly terminal **126.** The return pad has a large surface area pad with electrically conductive adhesive gel to give low impedance connection to the patient's skin for the duration of the surgery. The large surface area allows for small current density to avoid bums at the ESU return pad site. The return-pad-assembly cable includes an insulator over one or more internal conductors configured to carry current to the ESU. The return-pad-assembly cable is typically six feet long or longer and serves to allow the ESU to sit away from the operating room table. The return-pad-assembly terminal allows for termination of the return pad assembly with the ESU. In this regard, the return-pad-assembly terminal typically includes a plurality of (e.g., two) pins, and the return input includes a plurality of receptacles for receiving respective ones of the pins, to support electrical continuity monitoring of the ESU return pad when connected to the patient **104.**

The ESU **102** is typically left in the operating room at all times, but some setup occurs when the patient **104** is brought into the room. Typically, the patient is induced (put under anesthesia) after being brought into the operating room. After the patient is asleep, they are positioned on the operating table. This is typically when the ESU return pad **122** is attached to the patient's hip, thigh or other easily accessed open area away from the surgical site. The return pad tissue is prepped by shaving the patient's hair for a low impedance connection. The next step is typically draping the patient with sterile drapes. After the sterile operating field has been established, the pencil **112** is passed, using sterile technique, to the sterile field and plugged into the ESU. At this point, the person responsible for adjusting the proper settings for the ESU adjusts the settings where the surgeon desires and the unit is operated by the surgeon using the activation buttons on the ESU pencil or sometimes a foot activated switch.

As shown in FIG. 2, it is not uncommon for the patient to have other medical devices with leadwires such as neurodiagnostic electrodes **128,** which may be attached to the patient's skin. Undesirably, however, electromagnetic coupling **130** can occur (e.g., capacitively or inductively) between the pencil-assembly cable **114** and leadwires of other medical devices in proximity to each other. The electromagnetic coupling pathway may exist through the air between the pencil assembly-cable and leadwire of the neurodiagnostic electrode. At the location where the neurodiagnostic electrode makes contact with tissue, if the surface area of the electrode is small, such as the approximately 15 mm² surface area of a subdermal needle electrode, an electrothermal bum can easily occur. It should be noted that dangers from the mentioned electromagnetic coupling may not be limited to the described danger with neurodiagnostic electrodes.

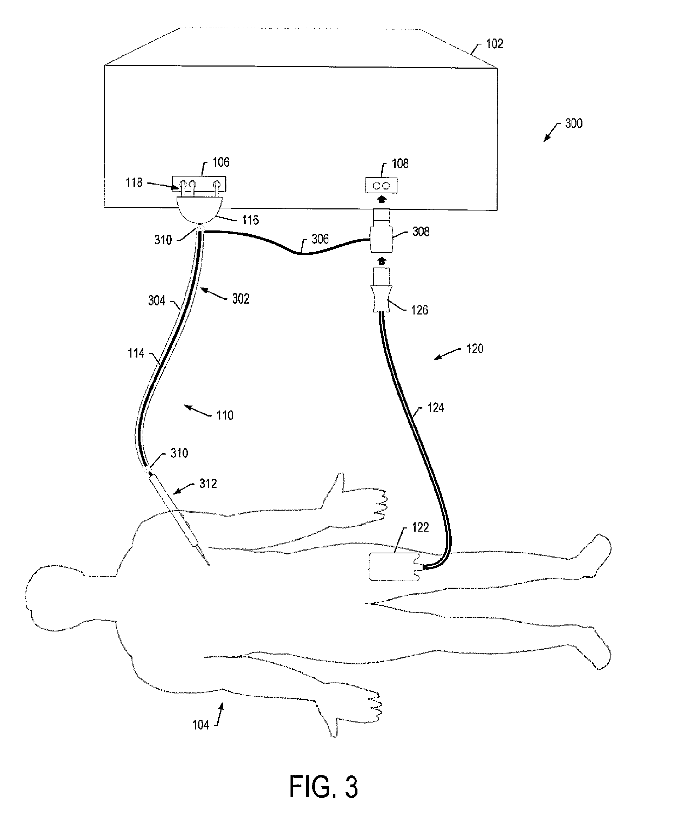

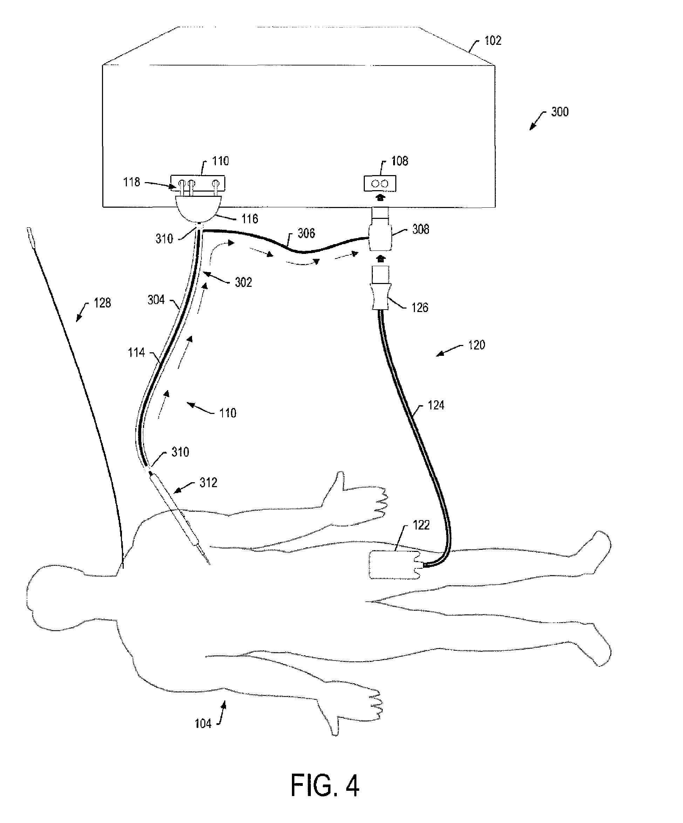

Example implementations of the present disclosure therefore provide an electromagnetic shield apparatus for ESUs **102** to shield other medical devices from potential electromagnetic coupling with emitted electromagnetic energy from the pencil-assembly cable **114.** FIGS. 3 and 4 illustrate a system **300** including an electromagnetic shield apparatus **302** according to one example implementation. As shown, the apparatus may include an electrically-conductive shield **304,** an insulated, low-impedance conductive cable **306,** a low-impedance, electrically-passive jumper **308** and end members **310** on respective opposing ends of the shield. The system may include a pencil **112** similar to before, or it may include a pencil **312** that functions as before but that may be modified to accommodate the apparatus of example implementations.

The shield **304** may cover and extend coaxially with the pencil-assembly cable **114** and its insulator and internal conductor(s), and the shield may be adhered to the pencil-assembly cable by the end members **310** of the apparatus to keep the shield from slipping. The shield may be electrically-connected to the return side **120** of the circuit. As shown, the passive jumper **308** may be coupled to and between the return-pad-assembly terminal **126,** and the low-impedance cable **306** may be coupled to and between the shield and passive jumper, without active circuitry or any alterations of the ESU circuitry function. In some examples, the passive jumper may include a plurality of pins receivable by respective receptacles of the ESU, and a plurality of receptacles for receiving respective pins of the return-pad-assembly terminal.

The apparatus **302** may be provided in a sterile pouch before setup in the operating room. Once the sterile field is established and the pencil-assembly cable **114** passed to the sterile field, the shield **304** may be applied over the pencil-assembly cable by the operating room staff. The passive jumper **308** may be connected at the ESU **102,** and the shield end members **310** may be attached to the pencil-assembly cable to secure the shield to keep it from sliding along the length of the shield. The shield may then remain in place during the surgery and should introduce no challenges to the normal form and function of the pencil-assembly cable.

With the electromagnetic shield apparatus **302,** the potential pathway for electromagnetic coupling between the neurodiagnostic electrode **128** leadwire and the pencil-assembly cable **114** may be reduced if not effectively removed. Instead, the shield **304** may pick up stray energy from the pencil-assembly cable and safely return the current directly to the return side **120** of the circuit, and thereby the ESU **102,** via the low-impedance pathway from the shield through the low-impedance cable **306** and passive jumper **308.** It should be noted that the apparatus of example implementations may prevent the dangerous electromagnetic coupling with any of a number of other medical devices including leadwires, and is not limited to neurodiagnostic electrodes.

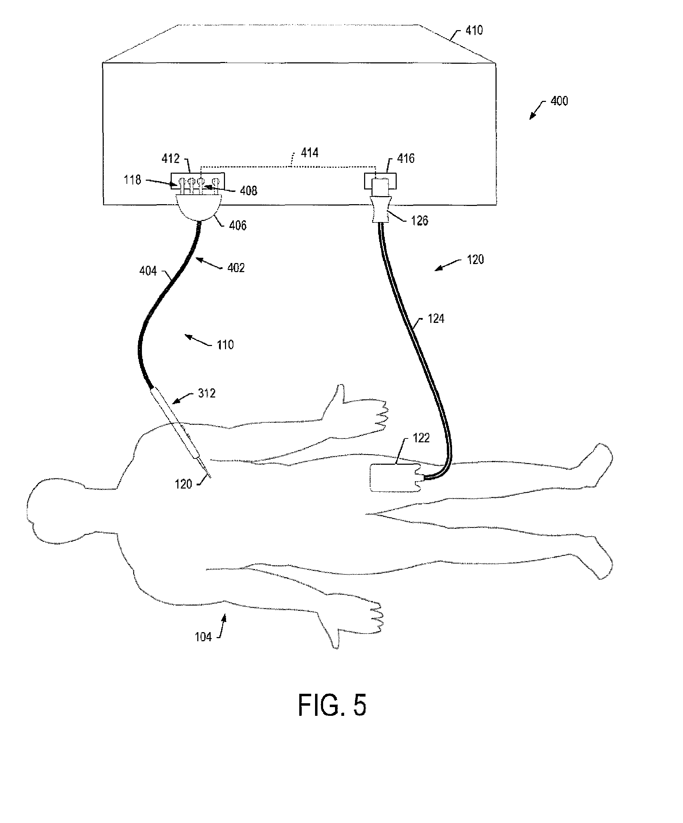

FIG. 5 illustrates a system **400** including an electromagnetic shield apparatus **402** according to another example implementation. As shown, the pencil assembly includes a pencil **312** similar to before, but its pencil-assembly cable **404** has a built-in shielding layer that covers the insulator and conductor(s) configured to carry current generated by the ESU **410,** and its pencil-assembly terminal **406** includes a fourth pin **408.** In one example, the shielding layer may extend at least partially into the pencil to thereby provide at least partial electromagnetic shielding of the pencil.

The ESU **410** of this example may have an active output **412** including a fourth receptacle for receiving the fourth pin, and a low-impedance conductive connection **414** providing a return pathway from the fourth receptacle to the return input **416.** As shown, then, when the pencil-assembly terminal is connected to the active output, the shielding layer of the pencil-assembly cable may be electrically connected to the fourth receptacle, and thereby the pencil-assembly cable may be electrically continuous with a low impedance pathway internal to the ESU. This example is similar to that of FIGS. 3 and 4, but builds the electromagnetic shielding into the pencil-assembly cable and ESU.

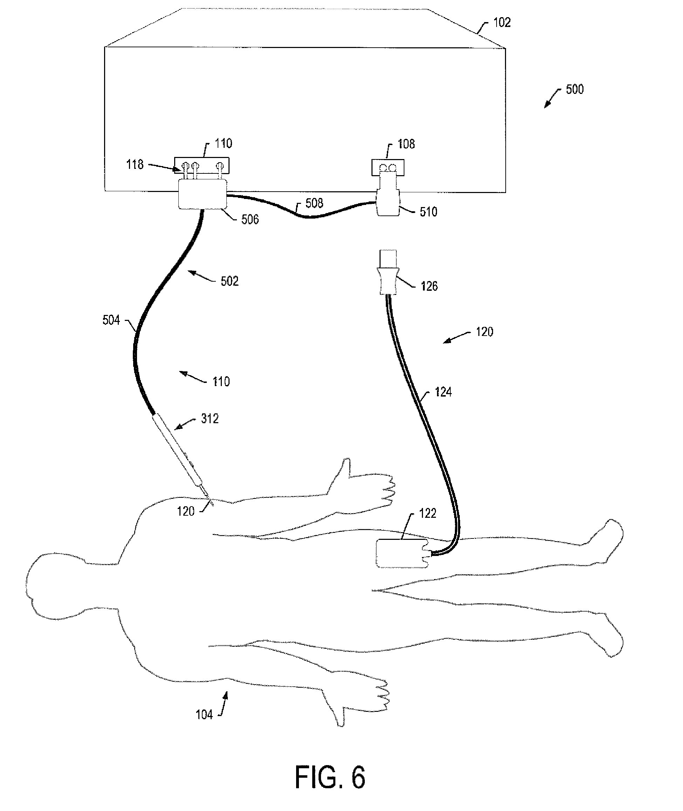

FIG. 6 illustrates a system **500** including an electromagnetic shield apparatus **502** according to yet another example implementation, which may include aspects of each of the examples of FIGS. 3 and 4, and FIG. 5. Similar to the example of FIG. 5, the pencil assembly of this example includes a pencil **312** and a pencil-assembly cable **504** having a built-in shielding layer that covers the insulator and conductor(s) configured to carry current generated by the ESU **102** (and that may extend at least partially into the pencil). Instead of the pencil-assembly terminal including an additional pin and ESU having an additional receptacle and low-impedance connection, however, the apparatus of this example may include pencil-assembly terminal **506** connected to a low-impedance cable **508** and passive jumper **510** connected to the shielding layer, similar to the example of FIGS. 3 and 4. In this regard, the built-in shielding layer may be electrically connected to the return side **120** of the circuit via the low-impedance cable and passive jumper without active circuitry or any alterations of the ESU circuitry function.

In the examples of FIGS. 5 and 6, the pencil-assembly cable **404, 504** may be provided in a sterile pouch to the operating-room staff. After a sterile field is established, the staff may pass the entire unit to the sterile field. The pencil **312** may be kept on the sterile field, while the connections to the ESU **102** may be passed off of the sterile field to be connected to the ESU as diagramed. The apparatus of these example implementations may operate during surgery as a standard pencil assembly and introduce no challenges to the normal form and function of the ESU.

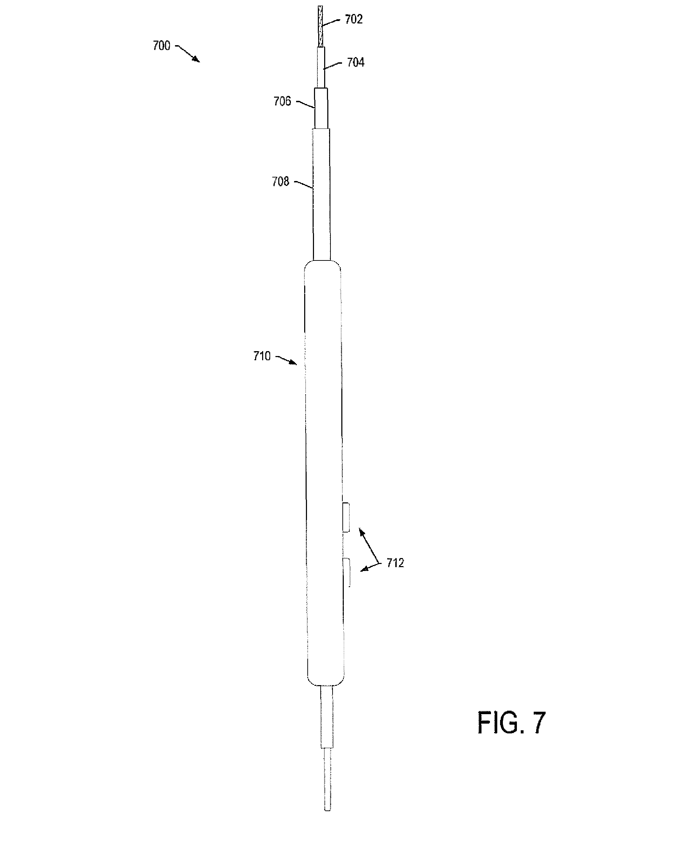

FIG. 7 illustrates a pencil **700** that according to one example implementation may correspond to pencil **112** or pencil **312.** As shown, the pencil includes a tip or one or more conductors **702,** and an insulator **704** covering or otherwise surrounding the conductor(s). This insulator may be the same as that of a typical pencil **112,** or according to example implementations, it may be thicker to decrease electromagnetic coupling between the conductor(s) and shield **304** or shielding layer. In the examples of FIGS. 5 and 6, the pencil may also include a built-in conductive shielding layer **706** to capture the stray electromagnetic coupled energy, and insulation **708** covering or otherwise surrounding the shield. The pencil may further include a base unit **710** including activation buttons **712** and/or other components for operating the pencil, which may be the same as that of the typical pencil.

Many modifications and other implementations of the disclosure set forth herein will come to mind to one skilled in the art to which these disclosure pertain having the benefit of the teachings presented in the foregoing descriptions and the associated drawings. Therefore, it is to be understood that the disclosure are not to be limited to the specific implementations disclosed and that modifications and other implementations are intended to be included within the scope of any appended claims. Moreover, although the foregoing descriptions and the associated drawings describe example implementations in the context of certain example combinations of elements and/or functions, it should be appreciated that different combinations of elements and/or functions may be provided by alternative implementations without departing from the scope of any appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. An electromagnetic shield apparatus for an existing electrosurgical unit (ESU) including a pencil assembly and return pad assembly, the pencil assembly including a pencil, pencil-assembly cable and terminal couplable to the ESU, and the return pad assembly including a return pad, return-pad-assembly cable and return-pad-assembly terminal couplable to the ESU, the electromagnetic shield apparatus comprising:
an electrically-conductive shield configured for placement over the pencil-assembly cable to thereby cover and extend coaxially with the pencil-assembly cable;
a low-impedance, electrically-passive termination jumper couplable to and between the return-pad-assembly terminal and ESU; and
an insulated, low-impedance conductive cable coupled to and between the electrically-conductive shield and termination jumper.

2. The electromagnetic shield apparatus of Claim 1, wherein the electrically-conductive shield is configured to reduce electromagnetic coupling of the pencil-assembly cable and a leadwire in proximity thereto.

3. The electromagnetic shield apparatus of Claim 1 or Claim 2, wherein the return-pad-assembly terminal includes a plurality of pins, and the ESU includes a plurality of receptacles for receiving respective ones of the pins, and
wherein the termination jumper includes a plurality of pins receivable by respective ones of the receptacles of the ESU, and a plurality of receptacles for receiving respective ones of the pins of the return-pad-assembly terminal.

4. The electromagnetic shield apparatus of any preceding claim further comprising:
end members on opposing ends of the electrically-conductive shield for adhering the electrically-conductive shield to the pencil-assembly cable.

5. A pencil assembly for an electrosurgical unit (ESU), the pencil assembly comprising:
a pencil;
a cable coupled to the pencil and including an insulator surrounding an inner conductor, and further including an electrically-conductive shielding layer covering the insulator and inner conductor; and
a terminal coupled to the cable and couplable to the ESU, the terminal including a plurality of pins receivable by respective receptacles of the ESU, the electrically-conductive shielding layer being coupled to one of the pins receivable by a respective one of the receptacles of the ESU,
wherein the respective one of the receptacles of the ESU is coupled by a low-impedance conductive connection to a return input of the ESU at which a return pad assembly is couplable.

6. The pencil assembly of any preceding claim, wherein the electrically-conductive shielding layer is configured to reduce electromagnetic coupling of the cable and a leadwire in proximity thereto.

7. An electromagnetic shield apparatus for an electrosurgical unit (ESU) having a return pad assembly including a return pad, return-pad-assembly cable and return-pad-assembly terminal couplable to the ESU, the electromagnetic shield apparatus comprising:
a pencil assembly comprising:
a pencil;
a pencil-assembly cable coupled to the pencil and including an insulator surrounding an inner conductor, and further including an electrically-conductive shielding layer covering the insulator and inner conductor; and
a pencil-assembly terminal coupled to the cable and couplable to the ESU;
a low-impedance, electrically-passive termination jumper couplable to and between the return-pad-assembly terminal and ESU; and
an insulated, low-impedance conductive cable coupled to and between the electrically-conductive shielding layer and termination jumper.

8. The electromagnetic shield apparatus of Claim 7, wherein the electrically-conductive shielding layer is configured to reduce electromagnetic coupling of the pencil-assembly cable and a leadwire in proximity thereto.

9. The electromagnetic shield apparatus of Claim 7 or Claim 8, wherein the return-pad-assembly terminal includes a plurality of pins, and the ESU includes a plurality of receptacles for receiving respective ones of the pins, and
wherein the termination jumper includes a plurality of pins receivable by respective ones of the receptacles of the ESU, and a plurality of receptacles for receiving respective ones of the pins of the return-pad-assembly terminal.
